Europäisches Patentamt

(19) European Patent Office

Office européen des brevets

(11) Publication number: **0 095 727**
**A1**

(12) # EUROPEAN PATENT APPLICATION

(21) Application number: 83105193.3

(22) Date of filing: 26.05.83

(51) Int. Cl.³: **A 61 N 1/04**, A 61 N 1/38

(30) Priority: 01.06.82 US 383353

(43) Date of publication of application: 07.12.83
Bulletin 83/49

(84) Designated Contracting States: CH DE FR GB IT LI NL
SE

(71) Applicant: PURDUE RESEARCH FOUNDATION, Hovde
Hall Purdue University, West Lafayette
Indiana 47907 (US)

(72) Inventor: Tacker, Willis A., Jr., 2901 Wilshire Avenue,
West Lafayette Indiana 47906 (US)
Inventor: Babbs, Charles Fredrick, 804 E. Cumberland
Avenue, West Lafayette Indiana 47906 (US)
Inventor: Bourland, Joe Dan, 606 Wilshire Avenue, West
Lafayette Indiana 47906 (US)
Inventor: Geddes, Leslie Alexander, 400 North River
Road Apt. 1724, West Lafayette Indiana 47906 (US)

(74) Representative: Wilhelm, Hans-Herbert, Dr.-Ing. et al,
Patentanwälte Dr.-Ing. Hans-Herbert Wilhelm Dipl.-Ing.
Hanjörg Dauster Gymnasiumstrasse 31B,
D-7000 Stuttgart 1 (DE)

(54) Method and apparatus for inserting a defibrillator electrode and defibrillator electrode.

(57) A novel defibrillator electrode assembly is provided, comprising a length of titanium mesh connected at a first end to an insulated wire and preferably having one or more barbs at the second end, the whole being contained within a cannula with a flattened end having slots to accommodate the barbs. The assembly is inserted, flattened end first, by way of an endoscope into the abdominal cavity and through an incision in the diaphragm/pericardium membrane into the pericardial sac and adjacent the epicardium, after which the cannula is withdrawn, leaving the electrode in place.

0095727

- 1 -

## Method and Apparatus for Inserting a Defibrillator Electrode and Defibrillator Electrode.

This invention relates to the control of cardiac ventricular fibrillation, and more particularly to an improved electrode system and a method for its implantation as a means of effecting ventricular defibrillation.

Ventricular fibrillation is a rapid, uncoordinated, and ineffectual arrhythmia of the heart which results in death in a matter of a few minutes after onset unless treated effectively. The most effective therapy is application of an electric shock of sufficient strength to depolarize most of the ventricular cells. Typically, for the hospitalized patient, the shock is applied by way of a pair of electrodes ("paddles") placed on the chest of the patient. An alternative effective therapy is implantation of an automatic defibrillator, designed to detect the onset of fibrillation and to apply the corrective shock to an implanted electrode system.

Two kinds of implanted electrode systems have heretofore been employed for controlling ventricular fibrillation: (1) a system having an epicardial electrode applied to the lower part of the epicardium (the outer wall of the heart) and a second electrode inserted transvenously above the heart into the superior vena cava (SVC) which is one of the large blood vessels, this being the so-called "apical-SVC electrode system"; and (2) a system in which both electrodes are on a catheter which is inserted transvenously; one electrode is in the right ventricle and the other is in the superior vena cava, this being the so-called "transvenous catheter-electrode system."

The apical-SVC electrode system contacts the left ventricle, which comprises 90% of the heart tissue, and is therefore efficient in distributing the defibrillating current throughout the ventricles, allowing a relatively small implanted pulse generator to be used. Unfortunately, major surgery is required to implant the apical electrode on the epicardium. The superior vena cava electrode is in contact with blood, and hence it gives rise to a potential problem of blood clot formation. For most candidate patients, major surgery is not tolerable except as a last resort; and for all patients, the formation of blood clots is a serious hazard.

The transvenous catheter electrode system has an advantage in being implantable without the need for thoracotomy -- i.e., without major surgery; but it is relatively inefficient in distributing the defibrillating current throughout the ventricles, with the result that the size of the implanted pulse generator is necessarily larger than for more efficient electrode systems. Another advantage of the transvenous catheter electrode system is that it provides high-amplitude, noise-free signals for monitoring ventricular activity; however, the signals it provides are from the right ventricle rather than the left ventricle, and thus it does not monitor the cardiac chamber having the most vital function. This electrode system also has the blood clotting problem of the apical-SVC electrode system, since with both systems, the electrode on the catheter is in contact with blood.

The electrode system of the present invention has all of the advantages of the existing systems, without any of their disadvantages. It avoids major surgery and thereby minimizes the problems of surgical shock, general anesthesia, disturbance and possible collapse of the lungs, damage to the heart, and infection. It avoids direct contact with the bloodstream, and thereby minimizes the problems of fibrosis, blood clot, and embolus formation, and associated problems of stroke and infarction. It avoids any need for a device inside the right ventricular chamber which may cause perforation and hemorrhage. It uses larger electrodes, allowing lower current density, lower voltages, and smaller generators, which are more easily implanted. The current distribution is better, reaching more of the ventricular tissues. It requires less equipment, personnel, and time for implantation; and is therefore substantially less expensive.

It is an object of the present invention to provide an improved method and means for controlling ventricular fibrillation.

Another object of the invention is to provide an improved electrode for the control of ventricular fibrillation that is implantable on the epicardium without major surgery.

A further object is to provide an electrode system for controlling ventricular fibrillation that avoids contact with the bloodsteam.

A still further object is to provide an implantable electrode system which, in combination with an implantable heart monitor and pulse generator, effects continuous surveillance of the patient's condition and prompt application of remedial measures
when ventricular fibrillation develops.

Another object is to install defibrillator
electrodes into the epicardium/pericardium juncture
through simple surgery with local anesthetic.

Other objects of the invention and its
advantages over the prior art will be apparent from the
following description.

In one of its several aspects, the present
invention provides an improved electrode, insertable
without major surgery between the epicardium and the
pericardium, for the control of ventricular
fibrillation.

In another aspect, the invention provides a
cannula assembly adapted for inserting the defibrillator
electrode between the epicardium and the pericardium,
comprising a cannula flattened and widened at its distal
end and a defibrillator electrode within said distal
end.

In a further aspect, the invention provides a
method for inserting a defibrillator electrode between
the epicardium and the pericardium, employing the
cannula assembly referred to above.

The objects of the present invention are
achieved by providing a defibrillator electrode having
one or more barbs at its distal end. The electrode is
received in a cannula having a flattened and widened
end, the barbs of the electrode being received in slots
in the flattened end of the cannula with the points of
the barbs being directed proximally. The insulated wire
connected to the electrode extends backwards from the
electrode through the cannula. The electrode is
positioned between the heart and the pericardium by
inserting the cannula bearing the electrode through an
incision in the juncture of the diaphragm and pericardium. The barbs do not engage tissue on insertion, but upon withdrawal of the cannula, the barbs on the electrode secure the electrode in position.

The accompanying drawings illustrate the invention and show preferred embodiments exemplifying the best mode of carrying out the invention as presently perceived. In such drawings:

Fig. 1 is a plan view of an electrode useful in the present invention;

Fig. 2 is an enlarged view in longitudinal cross section of the electrode of Fig. 1 at line 2-2;

Fig. 3 is a plan view of a cannula useful in the present invention;

Figs. 4 and 5 are cross-sectional views of the cannula of Fig. 3 at lines 4-4 and 5-5, respectively;

Fig. 6 is a longitudinal cross-sectional enlarged view of the cannula of Fig. 3 showing the electrode of Fig. 1 in place therein;

Fig. 7 is a frontal view of the human heart, partly in lateral cross section, showing an endoscope inserted through the abdomen with the cannula-electrode assembly of Fig. 6 in place in the pericardial space on the right side of the heart;

Fig. 8 is the same view as Fig. 7, with the cannula-electrode assembly of Fig. 6 in place in the pericardial space on the left side of the heart; and

Fig. 9 is the same view as Fig. 8, with the cannula and the endoscope removed, leaving the electrodes in place in the right and left pericardial spaces.

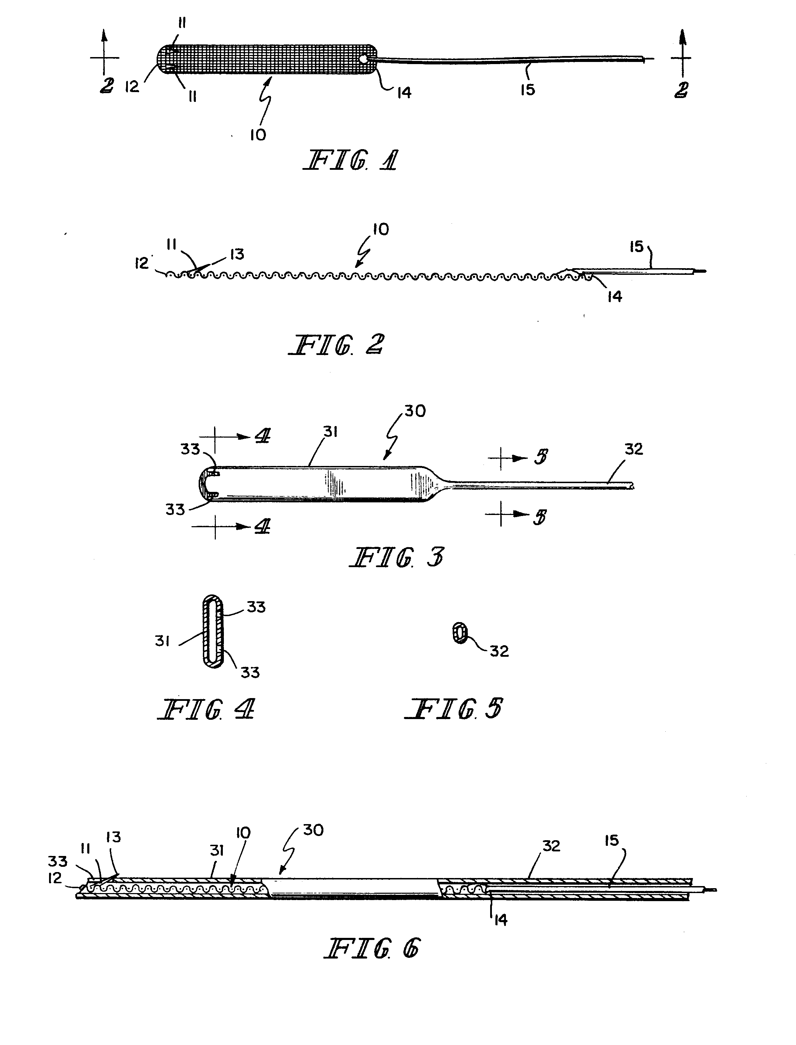

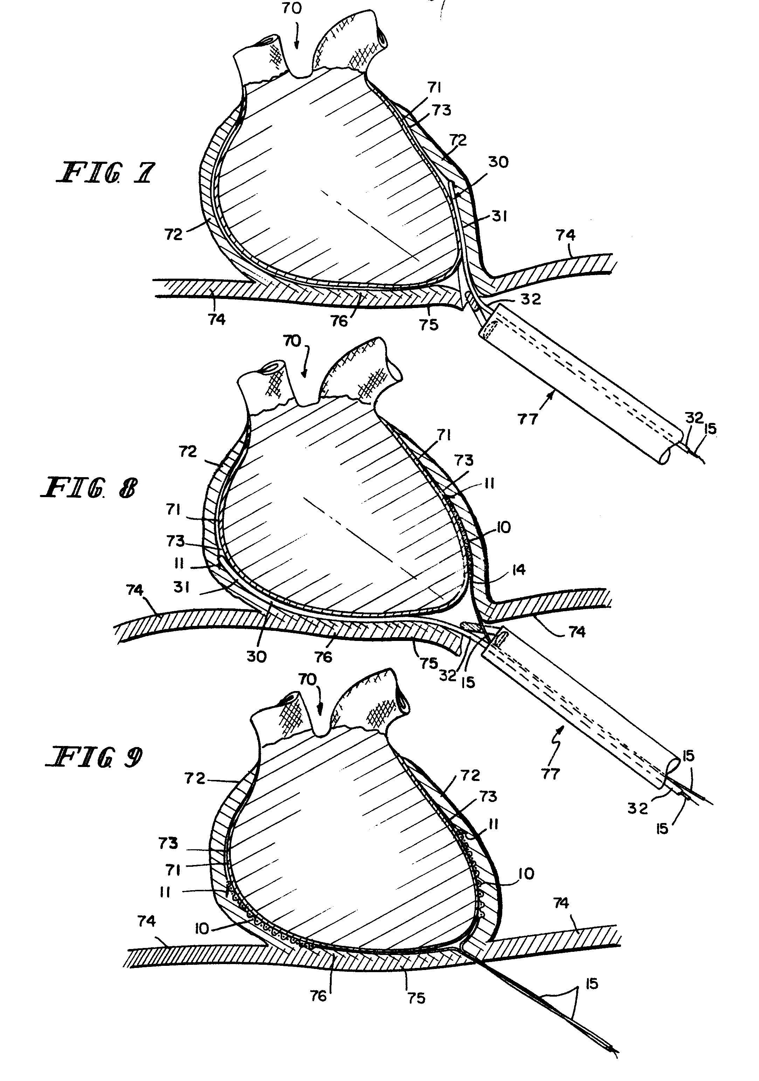

The electrode 10 of Fig. 1 is illustrated as a rectangular screen, suitably of convenient mesh-size for flexibility, measuring, for example, around 10 mm in width and 7 cm in length. Other shapes and forms may be used, if desired, such as a flexible, conducting ribbon.

The screen form is preferred, however, since, when inserted into the pericardial space, it undergoes tissue ingrowth and quickly becomes fixed in place. As an aid to implantation, the electrode 10 may desirably have one or more barbs 11 near its end 12 (the distal end as installed) on the side facing the pericardium, with their points (13 in Fig. 2) extending proximally, so that the electrode, when pushed into place, becomes affixed to the pericardium by a reverse motion, such as the withdrawal of the cannula as described below. Orientation of the barbs toward the heart should, of course, be avoided, since they would cause bleeding upon penetrating the heart wall, whereas they are less likely to cause a problem in penetrating the pericardium, which contains very few blood vessels. The electrode is suitably made of a conductive material that is inert to the body tissues, suitably stainless steel, tantalum, conductive plastic, or the like, preferably titanium. To the proximal end 14 of the electrode is attached an insulated lead wire 15, the insulation being suitably polyurethane, silicone, or other known tissue-compatible insulating materials. Similar lead wires are conventionally employed in implantable cardiac pacemakers, although in this instance the insulation must be designed for the higher voltages employed in defibrillation.

The size of the electrode represents a trade-off between the desired current density and the voltage to be employed. The use of an electrode of small area limits the voltage that can be applied to defibrillate owing to the risk of damaging the tissue beneath the electrode because of high current density. Moreover, large electrodes provide better distribution of current throughout the ventricles than small electrodes. On the other hand, the larger the electrode area, the more cumbersome it is to implant. A balancing of these factors is necessary. We find that the average current density should be between about 0.5 and about 1.5 ampere per square centimeter, preferably about one ampere per square centimeter. Suitable voltages range from about 200 to about 600 volts. Preferred voltage and current depend on implantable generator design. The electrode size may lie between about 5 and about 20 square centimeters, and the length-to-width ratio may be between about 2.5 and 10.

The voltages employed with our improved electrodes are substantially smaller than those required with the smaller prior art endocardial electrodes and catheter-based electrodes, the latter two typically using 1500 volts for an electrode area of 1.25 square centimeters. With our electrodes, the insulation requirements are less exacting and a smaller pulse generator can be used, allowing for easier implantation. In all cases, the defibrillating voltage is applied as a short pulse, ordinarily of the order of 2 to 5 milliseconds.

Fig. 2 illustrates the electrode 10 of Fig. 1 in longitudinal cross section at line 2-2, showing barb 11 near distal end 12 with barb 13 pointing proximally. Fig. 3 illustrates a cannula 30 suitable for inserting the electrodes of the present invention into the epicardial-pericardial space. The device is a tube, flattened at its distal end 31 and optionally flattened to some extent at its proximal end 32. (See cross-sectional views at lines 4-4, Fig. 4, and 5-5, Fig. 5, respectively). Slots 33 are provided in distal end 31 to accommodate and at least partially cover barbs 11 during insertion and to allow subsequent withdrawal of the cannula. Distal end 31 is of a length to accommodate electrode 10.

Fig. 6 shows cannula 30 in longitudinal cross section with electrode 10 lying in position within it, barbs 11 protruding slightly from slots 33 and lead wire 15 protruding from proximal end 32.

Cannula 30 can suitably be made of any substance that is compatible in the short term with tissue, stiff enough to be inserted through an endoscope into the pericardial space, and flexible enough for the electrode-bearing end to conform to the epicardial surface. Suitable substances include polyethylene, silicone, and polyether urethane.

The insertion and implantation of our novel electrode are shown in Figs. 7, 8, and 9.

Fig. 7 is a view, partly in section, of the heart 70 showing the outer surface 71 of the heart (the epicardium), the outer sac 72 enclosing the heart (the pericardium), the pericardial space 73 lying between them, the diaphragm 74 separating the thorax from the abdomen, the pericardium-diaphragm juncture 75 at which the two fuse into a single membrane, and an endoscope 76 having its distal end resting against membrane 75 beneath the heart. The endoscope is of conventional design having two barrels, one fitted with a light and optical means for observation, and the other open for insertion of operating devices. The endoscope is inserted into the abdominal cavity through an incision in the abdominal wall and is moved upward and rested against membrane 75 at a point beneath the left central portion of the heart. A knife is inserted through the operating barrel and an incision is made in membrane 75 of a size to admit the distal end 31 of cannula 30. The knife is withdrawn and cannula 30, with electrode 10 and lead wire 15 in place, is inserted through the operating barrel and pushed forward, being diverted to the right by deflector 77 and upward into the pericardial space on the right side of the heart as shown in Fig. 7. In this position, barbs 11 hook into the wall of the pericardium and retain electrode 10 in place when cannula 30 is withdrawn, as shown in Fig. 8.

A second electrode is implanted on the left side of the heart according to the same procedure, the endoscope being rotated 180° to cause deflector 77 to divert cannula 30 and electrode 10 toward the left side of the heart as shown in Fig. 8.

With the electrodes in place as shown in Fig. 9, a pulse generator and control unit (which are of known design) are implanted in a known way under the skin, usually of the abdomen or chest, and are connected internally to the lead wires 15. The device is then ready to function. Sensing devices in the control unit detect the onset of fibrillation and apply the necessary control pulses to the implanted electrodes, thereby restoring the normal heartbeat.

While we have described the invention with reference to certain specific embodiments, such embodiments are set forth as illustrative only and not by way of limitation. Numerous modifications will be apparent to those skilled in the art without departing from the spirit of the invention.

- 1 -

CLAIMS

1.      A method for inserting a defibrillator electrode between the heart and the pericardium which comprises inserting an endoscope through the abdominal wall into the abdominal cavity to a point adjacent the diaphragm and immediately beneath the heart, making an incision through the diaphragm and the pericardium with a knife introduced through the endoscope, withdrawing the knife, inserting through the endoscope and through the incision into the region between the epicardium and the pericardium an open-ended cannula bearing in its distal end a defibrillator electrode connected to an insulated wire extending backward to and emerging from the proximal end of the cannula, withdrawing the cannula while leaving the electrode in place, and withdrawing the endoscope.

2.      The method of claim 1 wherein said defibrillator electrode includes one or more barbs near its distal end adapted to catch into the pericardium.

3.      The method of claim 1 wherein said defibrillator electrode is made of titanium mesh.

4.      The method of claim 1 wherein said cannula is flattened and widened at its distal end to accommodate said defibrillator electrode.

5.      The method of claim 1 wherein two defibrillator electrodes are positioned laterally on opposite sides of the heart between the epicardium and the pericardium.

6.    A cannula assembly adapted for inserting a defibrillator electrode (10) between the epicardium and the pericardium, which comprises a cannula (30) flattened and widened at its distal end (31) to accommodate a defibrillator electrode (10), a defibrillator electrode (10) within said distal end (31) having one or more barbs (11) at its distal end (12) with points (13) directed proximally and resting in slots (33) in said cannula (30), and an insulated wire (15) connected to the proximal end (14) of said defibrillator electrode (10) and extending backward to and emerging from the proximal end (32) of said cannula (30).

7.    A defibrillator electrode (10) assembly comprising a portion of titanium mesh between about 5 cm and about 9 cm in length, and about 8 mm and about 20 mm in width, having one or more barbs (11) attached at one end with points (13) directed toward the body of the electrode (10), and an insulated wire (15) attached at the opposite end (14).

8.    An apparatus for inserting a defibrillator electrode (10) between the heart and the pericardium which comprises an endoscope for insertion through the abdominal wall into the abdominal cavity to a point adjacent the diaphragm and immediately beneath the heart, a knife introduced and withdrawn through the endoscope for making an incision through the endoscope for making an incision through the diaphragm and the pericardium, an open-ended cannula (30) for insertion through the endoscope and through the incision into the region between the epicardium and the pericardium, the open-ended cannula (30) bearing in its distal end (31) a defibrillator electrode (10) connected to an insulated wire (15) extending backward to and emerging from the proximal end (32) of the cannula (30), and means (11) for affixing the defibrillator electrode (10) to the pericardium leaving the electrode (10) in place when the cannula (30) and endoscope are withdrawn.

9.      The apparatus of claim 8 wherein the electode (10) affixing means includes one or more barbs (11) near the distal end (12) of the electrode (10) adapted to catch into the pericardium.

10.      The apparatus of claim 8 wherein said defibrillator electrode (10) is made of titanium mesh.

11.      The apparatus of claim 8 wherein said cannula (30) is flattened and widened at its distal end (31) to accomodate said defibrillator electrode (10).

*FIG. 1*

*FIG. 2*

*FIG. 3*

*FIG. 4*

*FIG. 5*

*FIG. 6*

FIG. 7

FIG. 8

FIG. 9

0095727

## PARTIAL EUROPEAN SEARCH REPORT

which under Rule 45 of the European Patent Convention shall be considered, for the purposes of subsequent proceedings, as the European search report

European Patent Office

### DOCUMENTS CONSIDERED TO BE RELEVANT

| Category | Citation of document with indication. where appropriate, of relevant passages | Relevant to claim | CLASSIFICATION OF THE APPLICATION (int. Cl. 3) |
|---|---|---|---|
| Y | US - A - 4 270 549 (MIROWSKI) <br><br> * Column 4, lines 33-41; column 5, lines 21-35; column 7, line 6 to column 8, line 39 * <br><br> -- | 6,7, 10 | A 61 N 1/04 <br> A 61 N 1/38 |
| Y | EP - A - 0 024 963 (CARDIOFRANCE) <br><br> * Page 3, line 36 to page 4, line 31; page 6, lines 3-29; page 9; figure 1 * <br><br> -- | 6,11 | |
| A | DE - A - 2 617 240 (MEDTRONIC) <br><br> * Page 13, last paragraph; page 14, paragraph 2; pages 15 to 18 * <br><br> -- <br> ./. | 6,8 | TECHNICAL FIELDS SEARCHED (Int. Cl. 3) <br><br> A 61 N <br> A 61 M |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 6-11
Claims searched incompletely:
Claims not searched: 1-5
Reason for the limitation of the search:

Method for treatment of the human or animal body by surgery or therapy (see article 52(4) of the European Patent Convention).

| Place of search | Date of completion of the search | Examiner |
|---|---|---|
| The Hague | 08-09-1983 | SIMON |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03 82'

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| A | DE - A - 2 643 956 (MIROWSKI) <br><br> * Page 10, last paragraph; page 12, last paragraph; page 20, last paragraph; page 21, first paragraph; page 23, first paragraph * <br><br> -- | 6-10 | |
| A | US - A - 4 058 128 (FRANK) <br><br> * Column 2, lines 33-52 * <br><br> -- | 6-9 | TECHNICAL FIELDS SEARCHED (Int. Cl.³) |
| A | IEEE PROCEEDINGS OF THE ANNUAL CONFERENCE ON ENGINEERING IN MEDICINE AND BIOLOGY, Houston, november 1968, vol. 10 <br> NEW YORK (US) <br> C.D. FIRRIS et al.: "Emergency cardiac pacing system", page 22A3. <br><br> * Page 22A3, paragraphs 3 and 4; figure 2 * <br><br> -- | 6,9 | |
| A | US - A - 4 181 123 (CROSBY) <br><br> * Column 2, lines 46-57 * <br><br> ---- | 8 | |

EPO Form 1505.3  06.78